**Europäisches Patentamt**

**European Patent Office**

**Office européen des brevets**

(11) Publication number: **0 150 246**
**B1**

(12) **EUROPEAN PATENT SPECIFICATION**

(45) Date of publication of patent specification: **12.04.89**

(21) Application number: **84100956.6**

(22) Date of filing: **31.01.84**

(51) Int. Cl.⁴: **A 61 B 5/04,** H 04 B 15/00

(54) Averaging method for elimination of periodic strays and a circuit arrangement for applying the method.

(43) Date of publication of application:
**07.08.85 Bulletin 85/32**

(45) Publication of the grant of the patent:
**12.04.89 Bulletin 89/15**

(84) Designated Contracting States:
**AT DE GB NL**

(56) References cited:
**DE-A-3 222 913**
**FR-A-1 321 021**
**FR-A-1 518 881**

**MEDICAL & BIOLOGICAL ENG., vol. 10, no. 10,
September 1972, pages 621-630, Peter
Peregrinus Ltd., GB; M. TEN HOOPEN et al.:
"Aspects of average response computation by
aperiodic stimulation"**

**MEDICAL & BIOLOGICAL ENG.,vol. 8, 1970,
pages 415-418, Pergamon Press, GB; H.J.
MARSONER et al.: "A method for triggering
evoked potentials by a certain component of
the background-activity"**

(73) Proprietor: **Instytut Psychiatrii i Neurologii
Al. Sobieskiego 1/9
Warszawa (PL)**

(72) Inventor: **Siarkiewicz Piotr H.
ul. Kulczynskiego 18 m. 4
Warszawa (PL)**
Inventor: **Zacharski, Bogdan W.
ul. Dereniowa 7 m. 22
Warszawa (PL)**

(74) Representative: **Lehn, Werner, Dipl.-Ing. et al
Hoffmann, Eitle & Partner Patentanwälte
Arabellastrasse 4
D-8000 München 81 (DE)**

(56) References cited:
**IEEE TRANSACTIONS ON BIOMEDICAL
ENGINEERING, vol. BME-26, no. 12, December
1979, pages 696-699, IEEE, New York, US; J.P.
ARY et al.: "A multiplexing system for
multichannel signal averaging of the visual
evoked response"**

**PATENTS ABSTRACTS OF JAPAN, vol. 7, no.
243 (E-207)1388r, 28th October 1983; & JP - A -
58 133 047**

Courier Press, Leamington Spa, England.

**EP  0 150 246  B1**

(56) References cited:

ICASSP 83, PROCEEDINGS, IEEE
INTERNATIONAL CONFERENCE ON
ACOUSTICS, SPEECH AND SIGNAL
PROCESSING, 14th-16th April 1983, Boston,
Massachusetts, vol. 1 of 3, pages 280-283, IEEE,
New York, US; H. RAUNER et al.: "Application
of a cepstral distance measure in evoked
potential processing"

JOURNAL OF PHYSICS E: SCIENTIFIC
INSTRUMENTS, vol. 9, no. 9, September 1976,
pages 783-786, GB; G. PALMER et al.: "Signal
averaging with a magnetic tape loop and
boxcar integrator"

IEEE TRANSACTIONS ON BIO-MEDICAL
ENGINEERING, vol. BME11, July 1964, pages
72-81; J.S. BENDAT: "Mathematical analysis of
average response values for nonstationary
data"

## Description

The present invention relates to an averaging method for elimination of periodic strays, e.g. strays of mains origin, and a circuit arrangement in a set-up measuring evoked responses for applying the method.

The averaging method for elimination of periodic strays, e.g. of mains origin, is suitable for use in analog-digital computers employing an averaging procedure. The averaging procedure elicits evoked responses from a set of signals $U_{zs}$ recorded from an examined subject. The set of signals $U_{zs}$ comprises: internal noise $U_{szw}$; individual evoked responses $U_{ow}$ from the examined subject; and periodic strays $U_{zp}$ induced in this subject from an environment comprising a source of the mentioned strays, the specified components of the set of signals being independent.

The above statements on the set of signals $U_{zs}$ can be presented by the following formula:

$$U_{zs}=U_{szw}+U_{ow}+U_{zp}.$$

The set of signals $U_{zs}$ does not comprise the component of apparatus noise $U_{sza}$ resulting from the operation of the apparatus which picks up and amplifies the set of signals $U_{zs}$, because the apparatus internal noise should be so small as to be neglected within the set of signals $U_{zs}$ during its continuous monitoring e.g. when observing this set on a monitor screen of an averaging computer. This noise can be recognised as the characteristic white noise, which due to its properties is non-synchronous with the aforementioned signals $U_{zs}$.

The mathematical basis of the averaging procedure has been described in a publication by J. S. Bendat: "Mathematical Analysis of Average Response Values for Non-stationary Data", IEEE Transactions on Bio-Medical Engineering, BME-11: 72—81, 1964, and its technical-clinical realization has been described by J. Kopeć: "Polish Computer ANOPS for Medical Research and its Clinical Application", Acta Physiologica Polonica, 21: 113—123, 1970.

Hitherto both the internal noise $U_{szw}$ of the examined subject and the periodic strays $U_{zp}$ were considered to be non-synchronous signals in relation to the individual evoked responses $U_{ow}$ and the averaging procedure could be described by the following formula:

$$\overline{U_{zs}}=\frac{1}{N}\left[\sum_{n=1}^{N}U_{ow}+\frac{1}{\sqrt{N}}\sum_{n=1}^{N}(U_{szw}+U_{zp})\right]$$

$$=\overline{U_{ow}}+\frac{1}{\sqrt{N}}(\overline{U_{szw}+U_{zp}})$$

$$\approx\overline{U_{ow}}$$

where

n=1, 2, 3, . . ., N; N at least several hundred.

As an example of the above-mentioned situation, with 256 sweeps of the averaging procedure, the square root of N equals sixteen, and with maximum amplitudes of the internal noise $U_{szw}$ and of the periodic strays $U_{zp}$ equal to 64 µV then their reduction as a result of the averaging procedure is sixteen-fold, thus their maximum averaged amplitudes $\overline{U_{szw}}$ and $\overline{U_{zp}}$ are not greater than 4 µV.

According to the above, the said averaging procedure, from the practical viewpoint, efficiently reduces the noise and the strays when the ratio of the maximum amplitudes of the said noise and strays to the least significant amplitudes of the said individual evoked responses is less than 20:1 and the reduction is proportional to the square root of the number of sweeps of the averaging procedure provided that the noise and strays are non-synchronous in relation to the individual evoked responses.

In unfavourable measuring conditions, even if the requirements regarding the said ratio are fulfilled, amplitudes of the periodic strays $U_{zp}$ may become comparable with amplitudes of the individual evoked responses $U_{ow}$ and the said strays may become synchronous in relation to the individual evoked responses $U_{ow}$, so that the averaged but nevertheless significant periodic strays $\overline{U_{zp}}$ can be superimposed on the averaged evoked response $\overline{U_{ow}}$. The superimposed signal may distort the averaged response to such an extent that its waveform is difficult to interpret.

The above-described situation can be described by the following formula:

$$\overline{U_{zs}}=\frac{1}{N}\left[\sum_{n=1}^{N}(U_{ow}+U_{zp})+\frac{1}{\sqrt{N}}\sum_{n=1}^{N}U_{szw}\right]$$

$$=(\overline{U_{ow}+U_{zp}})+\frac{1}{\sqrt{N}}\overline{U_{szw}}\cong\overline{U_{ow}}+\overline{U_{zp}}$$

where n=1, 2, 3, . . ., N; N at least several hundred.

As an example of the above-described situation, with 256 sweeps of the averaging procedure, the square root of N equals sixteen and where the maximum amplitudes of the internal noise $U_{szw}$ and of periodic strays $U_{zp}$ are equal to 64 $\mu V$, then the sixteen-fold reduction in the averaging procedure is effective only in respect of the averaged internal noise $U_{szw}$ resulting in its amplitude not exceeding 4 $\mu V$, while the averaged peridoic strays $U_{zp}$ behave as the averaged evoked response $U_{ow}$, are not reduced, and remain equal to 64 $\mu V$. In such a case, other known means for reduction of noise and various strays are commonly used, i.e. analog and/or digital filtering means. The known filter circuits may be divided into: low-pass filters—eliminating strays and higher frequency signals, high-pass filters—eliminating strays and lower frequency signals and band-stop filters—eliminating strays and signals of frequency equal to strays frequency. Each kind of the above-mentioned filters interferes with and distorts the original waveform of the signals $U_{zs}$. Interference and distortions introduced by the said filters cause elimination not only of strays but also signals of component frequencies comprised in the evoked responses, as the said responses usually comprise signals of frequencies comparable to the fundamental frequency of the strays.

The evoked response measuring set-ups, known to us, described and manufactured by such companies as: Nicolet Biomedical Co.—USA, Medelec Limited—Great Britain, Disa Elektronik A/S—Denmark, Technical University of Warsaw—Poland are only able to eliminate periodic strays by means of the said averaging procedure, and in the case where the periodic strays $U_{zp}$ and the individual evoked responses $U_{ow}$ are synchronous, can be supported by the said known filtering circuits.

An object of the invention is to provide a method and a circuit arrangement for substantially eliminating periodic strays in an averaging procedure while the original desired waveform is substantially undistorted.

According to one aspect of the invention, there is provided an averaging method for elimination of periodic strays in analog-digital computers employing an averaging procedure, in which method a set of signals $U_{zs}$ picked up by electrodes form an examined subject responding to external stimuli is processed to provide an averaged evoked response $U_{ow}$ characterised in that periodic strays $U_{zp}$ are eliminated from the set of signals $U_{zs}$ during the averaging procedure in proportion to the number N of sweeps of the averaging procedure resulting in at least an N-fold reduction of the amplitude of the strays by triggering the external stimuli and simultaneously the sweeps of the averaging procedure at such times that the phases of the periodic strays at two successive triggerings differ by a phase shift $\alpha$ within the range $120° \leq \alpha \leq 240°$.

According to another aspect of the invention, there is provided a timing circuit for an evoked response measuring set-up for applying an averaging method for strays elimination comprising a triggering circuit delivering common pulses which trigger successive sweeps of the averaging procedure in an averaging computer and simultaneously trigger external stimuli in transducers controlled by a stimulator characterised in that the triggering circuit is connected through a dividing circuit to a quartz oscillator circuit, in that the triggering circuit is driven by pulses of a highly stable frequency, which pulses are supplied by the dividing circuit and which frequency is determined by dividing the frequency of pulses supplied by the quartz oscillator circuit to the dividing circuit by a chosen integer P in the dividing circuit.

Construction of a timing circuit for the evoked response measuring set-up is one aspect of the invention. The circuit employing the averaging method for elimination of the periodic strays prevents the said measuring set-up from interfering with and distorting the original analog waveforms of the individual evoked responses $U_{ow}$ from the examined subject, and causes periodic strays $U_{zp}$ to be unambiguously eliminated from the averaged evoked responses $\overline{U_{ow}}$ in proportion to the number N of sweeps and thus the reduction is at least N-fold which means that the above-mentioned formula describing the averaged set of signals $\overline{U_{zs}}$ during the averaging procedure is as follows:

$$U_{zs} = \frac{1}{N} \left[ \sum_{n=1}^{N} U_{ow} + \frac{1}{N} \sum_{n=1}^{N} U_{zp} + \frac{1}{\sqrt{N}} \sum_{n=1}^{N} U_{szw} \right]$$

$$= \overline{U_{ow}} + \frac{1}{N} \overline{U_{zp}} + \frac{1}{\sqrt{N}} \overline{U_{szw}} \cong \overline{U_{ow}}$$

where n=1, 2, 3, ..., N; N at least several hundred.

As an example of the above situation, with 256 sweeps of the averaging procedure, the square root of N equals sixteen and the maximum amplitudes of the internal noise $U_{szw}$ and the periodic strays $U_{zp}$ are equal to 64 $\mu V$, then the sixteen-fold reduction in the averaging procedure is effective only in respect of the averaged internal noise $U_{szw}$ resulting in it having maximum averaged amplitudes not greater than 4 $\mu V$, while the averaged periodic strays $U_{zp}$ are reduced at least 256-fold leaving components with amplitudes not greater than 0.25 $\mu V$ in the set of the averaged signals and in an ideal case, when the periodic strays $U_{zp}$ and the individual evoked responses $U_{ow}$ are non-synchronous and number of N sweeps approaches infinity, the reduction value also approaches infinity and thus components of the averaged periodic strays $U_{zp}$ approach zero.

The aim of the invention has been achieved by developing an averaging method based on elimination of the periodic strays $U_{zp}$ from the set of signals $U_{zs}$ during the averaging procedure to a greater extent, i.e.

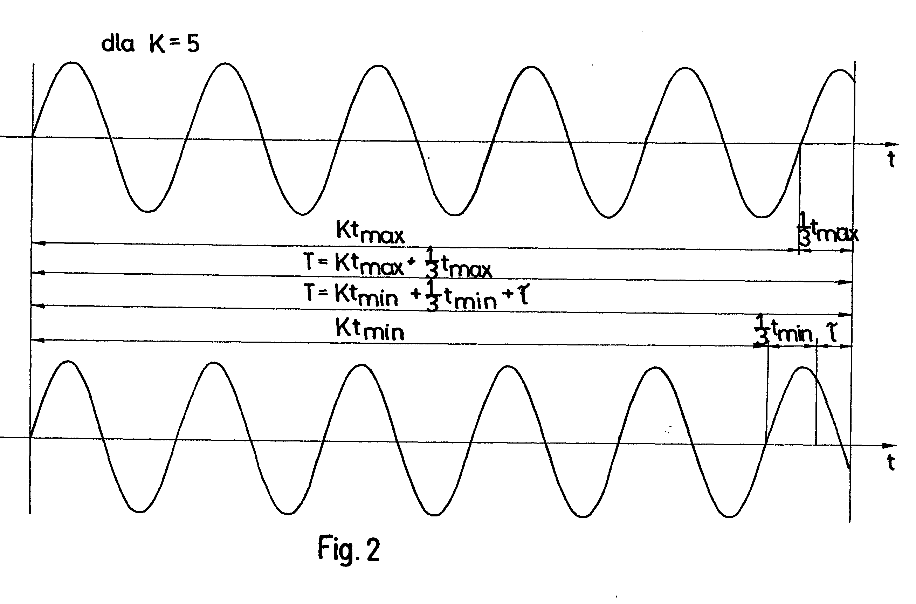

in proportion to the number N of sweeps of the averaging procedure, thus resulting in their at least N-fold reduction by introducing a phase shift by an angle $a$ in the range $120° \leq a \leq 240°$ related to the strays $U_{zp}$, which control periods of the external stimuli and initial phases of the periodic strays at the beginning of two successive periods of the external stimuli or alternately at the beginning of two successive sweeps of the averaging procedure differ by the value of the introduced phase shift i.e. by $a$, while the phase shift by $a$ secures permanent desynchronization between the external stimuli frequency 1/T and the frequency 1/t of the periodic strays as shown in Figure 2 and is determined from $T = const = Kt_{max} + \frac{1}{3}t_{max}$, where K is a natural integer comprised within the range

$$0 \leq K \leq \frac{t_n - 5\Delta t_n}{6\Delta t_n}.$$

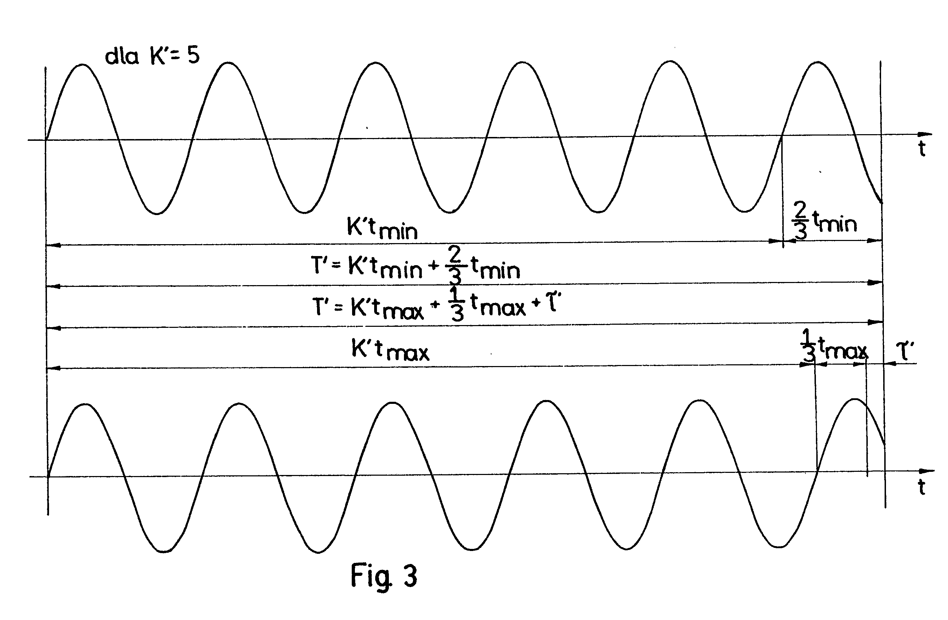

The aforementioned N-fold reduction can be also achieved by introducing permanent desynchronization between external stimuli frequency 1/T, and the frequency 1/t of the periodic strays $U_{zp}$ as shown in Figure 3 and is determined by

$$T' = const = K't_{min} + \frac{2}{3}t_{min},$$

where the K' is a natural integer within the range

$$0 \leq K' \leq \frac{t_n - 3\Delta t_n}{6\Delta t_n},$$

and the permanent desynchronization between the external stimuli frequency 1/T, and the frequency 1/t of the periodic strays $U_{zp}$ is chosen in such way that the period T' is a multiple of the period t of the periodic strays $U_{zp}$ i.e. $K(t_n + \Delta t_n)$ increased by $\frac{1}{3}(t_n + \Delta t_n)$ and by a time shift $\tau = 2\Delta t_n(K + \frac{1}{3})$, which two last together correspond to the phase shift $a$ or at the same time permanent desynchronization between the external stimuli frequency 1/T, and the frequency 1/t of the periodic strays $U_{zp}$ is achieved by introducing the period T' being a K' multiple of the period t of the periodic strays $U_{zp}$ i.e. $K'(t_n - \Delta t_n)$ increased by $\frac{2}{3}(t_n - \Delta t_n)$ and by a time shift

$$\tau' = \frac{1}{3}t_n - 2\Delta t_n(K' + \frac{1}{2}),$$

which two last together correspond to the phase shift $a$. A timing circuit for the evoked response measuring set-up for applying the averaging method for periodic strays elimination comprises a triggering circuit which is connected to a quartz osicllator circuit and by means of a dividing circuit is driven by high stability pulses of frequency P times less than the frequency of pulses supplied by the quartz oscillator circuit, and the frequency of the triggering pulses is computed by dividing the frequency $f_{gz}$ by a divisor P in the dividing circuit, which frequency can be calculated from the formula

$$f_{gz} = \frac{P}{(t_n + \Delta t_n)(K + \frac{1}{3})}$$

for the K derived according to Figure 2, which pulses are fed to the dividing circuit from the quartz oscillator circuit or the frequency 1/T, of the triggering pulses is a result of dividing the frequency $f_{gz}$ by a divisor P in the dividing circuit, which frequency may be calculated from the formula

$$f'_{gz} = \frac{P}{(t_n - \Delta t_n)(K' + \frac{2}{3})}$$

for the K' derived according to Figure 3 and pulses of this frequency are fed from the quartz oscillator circuit to the dividing circuit.

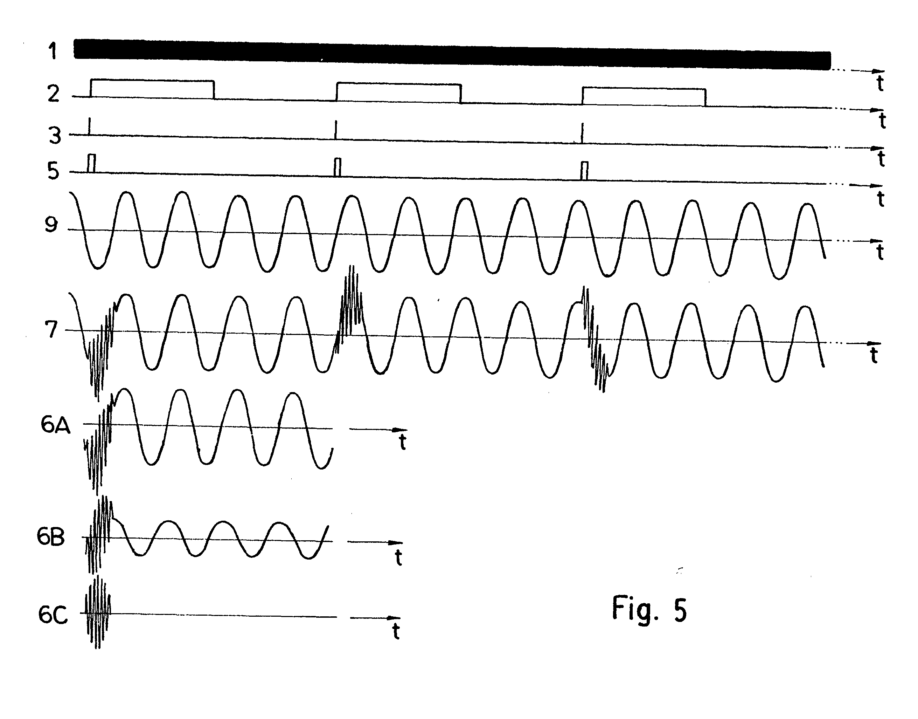

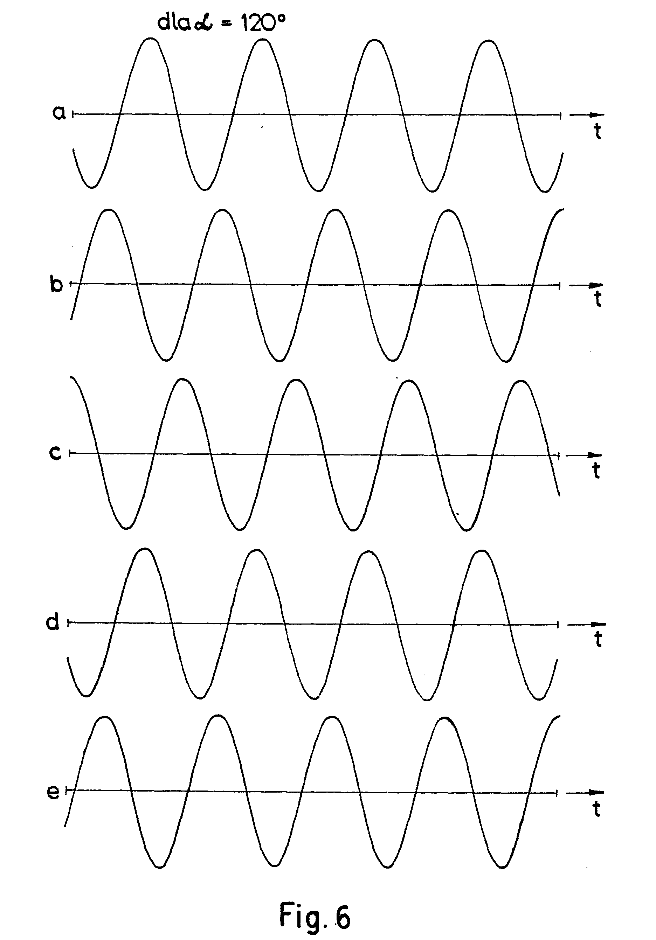

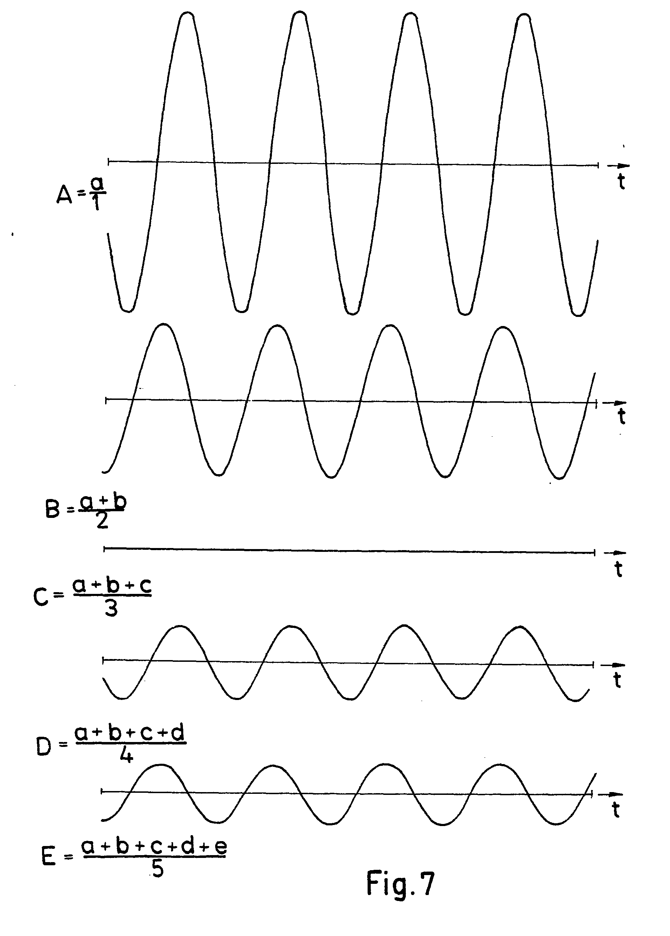

The above-described way of achieving the aim of the invention has the following advantages. Every consecutive external stimulus appearing after time T or T' causes every sweep of the averaging procedure to be delayed by the time shift $\tau$ or $\tau'$ respectively, which has the result that the aforementioned strays $U_{zp}$ comprised in the set of signals $U_{zs}$ are also shifted in time by $\tau$ or $\tau'$ in respect of every individual evoked response $U_{ow}$, which response is also comprised in the said set of signals $U_{zs}$, the relevant response is taken from the preceding period T or T' in respect of the following period T or T' respectively, as is shown in Figure 2 and Figure 3 respectively, and in Figures 5 and 6. The aforementioned time shift $\tau$ or $\tau'$ secures permanent desynchronization between the periodic strays and the external stimuli, and consequently between the strays and the individual evoked responses $U_{ow}$ and therefore the said strays are effectively eliminated in the averaging procedure in proportion to the number of N sweeps of the averaging procedure, as is illustrated in Figures 5 and 7. This means that amplitudes of the said strays are reduced at least N-fold where N is equal to the number of sweeps of the averaging procedure. The substantial advantage of the invented method is that the periods T or T' of the external stimuli are in each case certainly non-synchronous with the periodic strays $U_{zp}$, e.g. of mains origin, thanks to the introduced time shift $\tau$ or $\tau'$, which shift is dependent on the current frequency of the periodic strays $U_{zp}$, the frequency $1/t$ being in the range

$$\frac{1}{t_{max}} < \frac{1}{t} < \frac{1}{t_{min}},$$

which parameters and relations are only used for calculating the frequency $f_{gz}$ or $f'_{gz}$ respectively, whereas during the averaging procedure of the said set of signals $U_{zs}$ the said parameters of the strays need be neither examined nor checked.

Application of the averaging method for periodic strays elimination and the timing circuit for the evoked response measuring set-up for applying the method is of particular importance for electrophysiological examinations determining the degree of conduction loss of nervous paths between a sense organ and a corresponding reception region of the cortex. Under unfavourable measuring conditions during a reception of e.g. brainstem auditory evoked potentials (individual evoked responses $U_{ow}$), when the averaging procedure elicits from an electroencephalogram (internal noise $U_{szw}$) the aforementioned individual evoked responses picked up from a scalp (examined subject) activated by acoustic stimuli (external stimuli), there is very often met a situation in which the averaging procedure may also elicit remaining components of strays of mains origin together with the individual evoked responses belonging to the set of signals $U_{zs}$, so that the strays are in synchronism with sweeps of the averaging procedure. The timing circuit according to the invention prevents in every case the appearance of the said synchronism.

For a better understanding of the invention, and to show how the same may be carried into effect, reference will now be made, by way of example, to the accompanying drawings, in which:

Figure 1 illustrates relations between angle and time parameters of sinusoidal periodic strays taking into consideration frequency tolerance of the said strays;

Figures 2 and 3 illustrate the relationship of the period T of the external stimuli in respect of periods t of the periodic strays taking into consideration a way of achieving frequency desynchronization of the said strays in respect to the said stimuli;

Figure 4 shows a block diagram of the evoked response measuring set-up employing the averaging method for periodic strays elimination;

Figure 5 shows electric signals related to the block diagram of Figure 4;

Figure 6 illustrates successive signals of the periodic strays taken to the averaging procedure; and

Figure 7 illustrates successive sweeps of the averaging procedure.

In Figures 6 and 7 the strays signal is shown alone which represents the desynchronized periodic strays in respect of successive pulses, which trigger successive sweeps of the averaging procedure and trigger the external stimuli which correspond to the sweeps and are correlated through the examined subject with the individual evoked responses, which responses and the internal noise are omitted for simplicity of the diagram. The internal noise has also been omitted in Figure 5 for clearance of signals 6A, 6B and 6C representation.

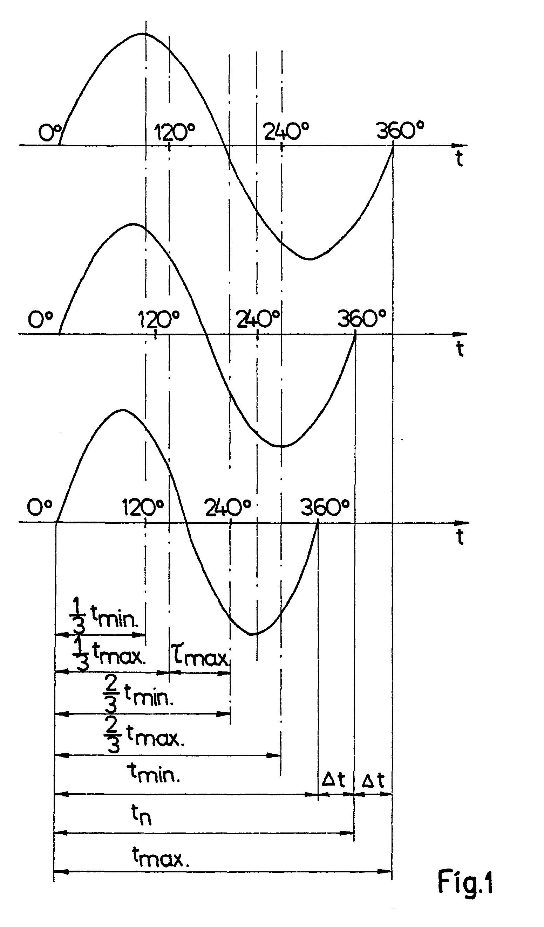

The method of periodic strays elimination according to the invention is based on the permanent desynchronization of the frequency $1/t$ of the said strays in respect to the frequency $1/T$ of pulses which trigger successive sweeps of the averaging procedure after every external stimulus. A triggering pulse originates both external stimulus and a sweep of the averaging procedure. The desynchronization between the frequency $1/t$ of the said strays and successive sweeps of the averaging procedure is based on the fact that K periods t, $\frac{1}{3}$ of period t as in Figure 2 or $\frac{2}{3}$ of period t as in Figure 3 and the time shift $\tau$ determine every period T between two successive external stimuli. The said time shift $\tau$ is in the range $0 < \tau < \tau_{max}$ and it corresponds to the phase shift $\alpha$. Because the said strays elimination is to be proportional to the number N of sweeps of the averaging procedure, the phase shift is within the range $120° \leq \alpha \leq 240°$. The said phase shift concerns the strays e.g. of mains origin, which strays appear in the next sweep of the averaging procedure shifted by $\alpha$ as compared with the preceding one. The value $1/t$ is the current value of mains frequency which may be comprised within limits of admissible tolerance determined by national regulations. According to Figure 1 the mains frequency $1/t$ must be comprised within the following limits

$$\frac{1}{t_{max}} \leq \frac{1}{t} \leq \frac{1}{t_{min}}$$

because $t_{min} = t_n - \Delta t_n$ and $t_{max} = t_n + \Delta t_n$, where $\pm\Delta t_n$ expresses the admissible tolerance of the said nominal mains frequency $1/t_n$.

An employment of the aforementioned method introduces a fixed value for a single value of K for a given period T of the external stimuli, which period may be described by the following formula as in Figure 2:

$$T=const=Kt_{max}+\tfrac{1}{3}t_{max}=Kt_{min}+\tfrac{1}{3}t_{min}+\tau$$

from which one may find the time shift as $\tau=2\Delta t_n(K+\tfrac{1}{3})$. The formula determines the time interval, dependent on K and being a difference between a maximum and a minimum period which is a result of the admissible tolerance $\pm\Delta t_n$ of the nominal period $t_n$ of the periodic strays e.g. of mains origin. From Figure 1, one may conclude that the maximum time shift $\tau_{max}$ corresponding to the phase shift range $120°\le\alpha\le240°$ represents a difference between respective parts of the admissible maximum and minimum periods of the periodic strays e.g. of mains origin, i.e.

$$\tau_{max}=\tfrac{2}{3}t_{min}-\tfrac{1}{3}t_{max}=\tfrac{1}{3}t_n-\Delta t_n.$$

From the above it is evident that the time shift $\tau$ must be within the range $0<\tau<\tfrac{1}{3}t_n-\Delta t_n$.

The averaging method of the periodic strays elimination according to the invention allows them to be reduced proportionally to N sweeps of the averaging procedure when the phase shift is within the range $120°\le\alpha\le240°$. To keep the above correct one needs to calculate K as a number of periods t of the periodic strays comprised in the time interval which is the period T of the external stimuli.

The averaging method for the periodic strays elimination requires period T to be equal or greater than $\tfrac{1}{3}t_{max}$ and the time shift $\tau$ to be less than $\tfrac{1}{3}t_n$ decreased by $\Delta t_n$ i.e. $T\ge\tfrac{1}{3}t_{max}$ and $\tau<\tfrac{1}{3}t_n-\Delta t_n$. Substituting $Kt_{max}+\tfrac{1}{3}t_{max}$ for T and $2\Delta t_n(K+\tfrac{1}{3})$ for $\tau$ in the preceding inequalities, the inequalities become

$$Kt_{max}+\tfrac{1}{3}t_{max}\ge\tfrac{1}{3}t_{max}$$

and

$$2\Delta t_n(K+\tfrac{1}{3})<\tfrac{1}{3}t_n-\Delta t_n.$$

Solving the set of inequalities in respect to K, one finds that K is comprised within the interval

$$0<K<\frac{t_n-5\Delta t_n}{6\Delta t_n}.$$

The method of the periodic strays elimination introduces a fixed value of the period T for a given value of K and the K has to be a natural integer i.e. K=0, 1, 2, 3, . . . .. Determining the interval of values of K has been necessary since if K were greater than

$$\frac{t_n-5\Delta t_n}{6\Delta t_n},$$

the phase shift would result in angle $\alpha$ greater than 240° and the N-fold reduction of the periodic strays would not be maintained.

The alternative employment of the aforementioned method for the periodic strays elimination is the introduction of another period T' of the external stimuli as is shown in Figure 3. The period T' of the external stimuli is described by the following formula as in Figure 3:

$$T'=const=K't_{min}+\tfrac{2}{3}t_{min}=K't_{max}+\tfrac{1}{3}t_{max}+\tau',$$

from which the result is that the time shift is

$$\tau'=\tfrac{1}{3}t_n-2\Delta t_n(K'+\tfrac{1}{2})$$

and it simultaneously determines the time interval resulting from the difference between a minimum and a maximum period associated with the admissible tolerance $\pm\Delta t_n$ of the nominal period $t_n$ of the periodic strays e.g. of mains origin, depending on the value of K' used. According to the above T' and $\tau'$ are modified as compared with T and $\tau$ respectively. The K, which in this case will be denoted as K', must be also modified. Determining the range of K' values the condition must be fulfilled that the period T' must be equal or greater than $\tfrac{2}{3}t_{min}$, while the time shift $\tau'$ must be greater than zero i.e. $T'\ge\tfrac{2}{3}t_{min}$ and $\tau'>0$. Substituting $K't_{min}+\tfrac{2}{3}t_{min}$ for T' and

$$\tfrac{1}{3}t_n-2\Delta t_n(K'+\tfrac{1}{2}) \text{ for } \tau'$$

in the above inequalities results in the following:

7

$$K't_{min} + \tfrac{2}{3}t_{min} \geq \tfrac{2}{3}t_{min}$$

and

$$0 < \tfrac{1}{3}t_n - 2\Delta t_n(K' + \tfrac{1}{2}).$$

From these inequalities the result is that the K' in this case lies within the range

$$0 < K' < \frac{t_n - 3\Delta t_n}{6\Delta t_n}.$$

In this case, the employment of the aforementioned averaging method for periodic strays elimination also introduces a fixed value of the period T' for a given K' and the K' must be a natural integer i.e. K'=0, 1, 2, 3, . . .. Determining the above range of values of K' was also necessary, because if the K' in this case were greater than

$$\frac{t_n - 3\Delta t_n}{6\Delta t_n},$$

the phase shift would be of angle α less than 120° and the N-fold reduction of the periodic strays would not be maintained.

To exemplify the averaging method for periodic strays elimination in Figures 5, 6 and 7 the ideal phase shift of angle α=120° is introduced and for better illustration of the periodic strays reduction in Figure 7, two-fold magnification of the amplitude scale in relation to Figure 6 is introduced. Besides, the desynchronized sinusoidal strays taken together with the internal noise $U_{szw}$ and the individual evoked response $U_{ow}$ during each sweep of the averaging procedure are shown in Figures 5 and 6, and they all are repeated in each successive time interval between every two successive external stimuli of frequency 1/T or 1/T', which time intervals correspond to the sweeps of the averaging procedure. The said sweeps of the averaging procedure are shown in Figure 7, where the waveform "A" represents the first sweep, the waveform "B" represents the second sweep and the waveform "C" represents the third sweep of the averaging procedure and during the third sweep the first complete reduction of the periodic strays takes place and further the waveforms "D" and "E" are the results of successive sweeps of the averaging procedure. In the case of phase shift by α=120°, complete reduction of the periodic strays takes place after every third sweep of the averaging procedure, which sweeps are counted starting from the waveform "A" as the first sweep.

Description of the averaging method for periodic strays elimination, in practical cases, requires a detailed discussion which uses some example data, since the said periodic strays may be in particular of mains origin of various values of nominal frequency $1/t_n$ depending on the place and examination conditions and may be e.g. 50 Hz, 60 Hz, 400 Hz etc.

For example, the mains may be a source of the periodic strays in form of a sine wave signal of the nominal frequency $1/t_n=50$ Hz with the admissible tolerance $\pm\Delta 1/t_n = \pm 1$ Hz which means that the period t of the said strays at a given moment of the examination procedure may be of any value within the tolerance range $t_{min}=19.6$ ms≤t≤20.4 ms=$t_{max}$. Next a fixed value of the period T of the external stimuli is to be assumed, as an example T=100 ms, and next the value is to be corrected in respect to values of T or T' being calculated from the aforementioned formulae, and next the correction is to be achieved by determining the value of K or K' resulting from the minimum value of the period $t_n$ of the strays of mains origin and of the fixed value of T'. Thus

$$K = \frac{T}{t} = \frac{100 \text{ ms}}{20 \text{ ms}} = 5,$$

subject to simultaneous checking against the condition:

$$K < \frac{t_n - 5\Delta t_n}{6\Delta t_n} = \frac{20 \text{ ms} - 5 \times 0.4 \text{ ms}}{6 \times 0.4 \text{ ms}} = 7.5.$$

The assumed value of K=5 determines the correct value of the period T, which in this case is:

$$T = const = Kt_{max} + \tfrac{1}{3}t_{max} = 5 \times 20.4 \text{ ms} + \tfrac{1}{3}20.4 \text{ ms} = 108.8 \text{ ms}.$$

The corrected value of T=108.8 ms i.e. of the time interval between successive external stimuli directly determines the frequency of pulses triggering the sweeps of the averaging procedure, which frequency is:

$$1/T \cong 9,191176 \cong 9.192 \text{ Hz}.$$

The value is approximated by rounding it up, which makes the frequency 1/T always slightly greater than the minimum required frequency and therefore the above-described condition on α (that it should be within the aforementioned phase range) is always fulfilled. Having determined the frequency of triggering pulses to be 9.192 Hz, the frequency $f_{gz}$ of the quartz oscillator circuit is set to be of such a value that the condition for angle α to be comprised within the aforementioned phase interval is fulfilled. The frequency $f_{gz}$ is obtained by multiplying a chosen integer P by the frequency 1/T. The chosen integer P should be a multiple of at most the three numbers: 2, 3 and 5. Such an approach allows for applying TTL IC's of series 7490, 7492, 7493. Taking $P=10^5$ and having the frequency of the triggering pulses 1/T=9.192 Hz, the frequency $f_{gz}$ is P1/T=0.9192 MHz. For quartz oscillator circuits a typical tolerance but only of the positive sign may be accepted, as the positive value always increases the nominal frequency $f_{gz}$ by $\Delta f_{gz}$ which makes the period T shorter and the condition that α be within the above-described phase shift range is fulfilled.

According to the above, the nominal frequency $f_{gz}$ of the quartz oscillator circuit may be calculated with the formula

$$f_{gz} = \frac{P}{(t_n + \Delta t_n)(K + \frac{1}{3})}$$

for K of Figure 2, or

$$f'_{gz} = \frac{P}{(t_n - \Delta t_n)(K' + \frac{2}{3})}$$

for K' of Figure 3.

The above aforementioned averaging method for periodic strays elimination enables design of the below-described evoked response measuring set-up, which first of all comprises a timing circuit for producing the period T or T' of the external stimuli which in each case is non-synchronous with the periodic strays e.g. of mains origin.

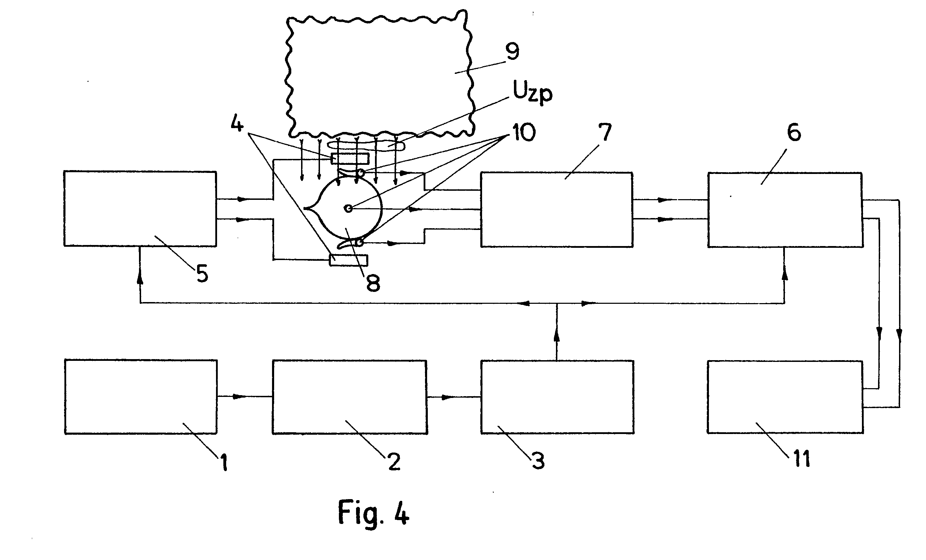

The set-up for measuring evoked responses, in particular brain-stem auditory evoked responses, for applying the above-described method is shown in Figure 4 and comprises a quartz oscillator circuit 1 for generating pulses of frequency $f_{gz}$ or $f'_{gz}$, frequency divider circuit 2 with a divisor P providing pulses of frequency

$$1/T = \frac{f_{gz}}{P}$$

or

$$1/T' = \frac{f'_{gz}}{P},$$

a circuit 3 for triggering successive external stimuli in transducers 4 through a stimulator 5 and for triggering simultaneously associated successives sweeps of the averaging procedure in an averaging computer 6, the stimulator 5 with transducers 4 being together the source of the external stimuli e.g. acoustic stimuli, an instrumentation amplifier circuit 7 for amplifying the set of signals $U_{zs}$, which signals are obtained from a subject 8 together with the periodic strays $U_{zp}$ originated from an environment 9 in which the said periodic strays of frequency 1/t are spread and the signals are picked up by means of electrodes 10 of Ag/AgCl type, the averaging computer 6 performing the averaging procedure of the said set of signals, and a memory and display block 11 for storing and displaying results of the averaging procedure.

Operation of the evoked response measuring set-up for applying the averaging method for periodic strays elimination is described below.

Pulses of the frequency $f_{gz}$ or $f'_{gz}$ produced by the quartz oscillator circuit 1 are directly sent to the frequency divider circuit 2, where the frequency $f_{gz}$ or $f'_{gz}$ is divided by the divisor P. At the output of the said circuit 2, pulses of frequency

$$1/T = \frac{f_{gz}}{P}$$

or

$$1/T' = \frac{f'_{gz}}{P}$$

are obtained, which pulses through an interfacing circuit comprised in the triggering circuit 3 are formed to actuate a triggering input of the stimulator 5 and a triggering input of the averaging computer 6. The said triggering pulses of frequency 1/T or 1/T' are fed through the circuit 3 to the stimulator 5 which causes generation of the external stimuli e.g. acoustic stimuli in acoustic transducers 4, from which transducers the stimuli reach the examined subject 8, which subject may be e.g. a human. The stimulator 5 e.g. an acoustic stimulator, after being triggered by a pulse from the triggering circuit 3 produces acoustic stimuli of appropriate loudness level as required. In the example of brain-stem auditory evoked responses, the phono stimulator 5 produces external stimuli of rectangular waveform resulting in a pattern effect in the acoustic transducers, while for the traditional auditory evoked responses the phono stimulator 5 produced the external stimuli in form of the acoustic stimuli but of sinusoidal function giving the effect of pure sound. The examined subject 8 is stimulated by the external stimuli in form of acoustic stimuli delivered by the acoustic transducers 4 which pulses through ear and audible nervous path produces an electrophysiological reaction in the pertinent regions of the cortex. The electrophysiological response corresponds to the individual evoked response $U_{ow}$ belonging to the set of signals $U_{zs}$. The set of signals $U_{zs}$ (which comprises the individual evoked responses $U_{ow}$, the internal noise $U_{szw}$ in this case being called an electroencephalogram and the periodic strays $U_{zp}$ originated from the environment 9 provided with mains supply network) are picked up by electrodes 10 from the scalp of the examined subject 8. The said set of signals is fed to the differential inputs of the circuit 7—two channel instrumentation amplifier. The said circuit 7 in each of its channels amplifies the said set of signals $U_{zs}$ to match its output voltage with the voltage range of analog inputs of the averaging computer 6. Each triggering pulse starts a successive sweep of the averaging procedure for the set of signals $U_{zs}$.

The averaged set of signals $\overline{U_{zs}}$ equals the averaged evoked response $\overline{U_{ow}}$. The product of the averaging procedure of the set of signals $U_{zs}$ in the averaging computer 6 is sent from the averaging computer 6 to the block 11 (memory and display) to provide the possibility for the future display and selection of the result and to provide data for further statistical analysis with the use of a large digital computer.

The method is of particular significance in the case of electrophysiological examinations, which determine a degree of deterioration in the nerve conduction paths linking a sense organ and an associated reception area of the cortex, and especially during the reception of brain-stem evoked responses.

**Claims**

1. An averaging method for elimination of periodic strays in analog-digital computers employing an averaging procedure, in which method a set of signals $U_{zs}$ picked up by electrodes (10) from an examined subject (8) responding to external stimuli is processed to provide an averaged evoked response $\overline{U_{ow}}$ characterized in that periodic strays $U_{zp}$ are eliminated from the set of signals $U_{zs}$ during the averaging procedure in proportion to the number N of sweeps of the averaging procedure resulting in at least an N-fold reduction of the amplitude of the strays by triggering the external stimuli and simultaneously the sweeps of the averaging procedure at such times that the phases of the periodic strays at two successive triggerings differ by a phase shift α within the range 120°≤α≤240°.

2. An averaging method according to claim 1, characterized in that the phase shift α is secured by permanent desynchronization between the external stimuli of frequency 1/T and the periodic strays of frequency 1/t and said desynchronization is determined by $T=Kt_{max}+\frac{1}{3}t_{max}$, where K is a natural integer within the range

$$0 \leq K \leq \frac{t_n - 5\Delta t_n}{6\Delta t_n}$$

$t_n$ is a nominal period of the periodic strays, $\Delta t_n$ is an admissible tolerance of $t_n$ and $t_{max}=t_n+\Delta t_n$.

3. An averaging method according to claim 1 characterized in that the phase shift α is secured by permanent desynchronization between the external stimuli of frequency 1/T and the periodic strays of frequency 1/t and said desynchronization is determined by $T'=K't_{min}+\frac{2}{3}t_{min}$, where K' is a natural integer within the range of

$$0 < K' < \frac{t_n - 3\Delta t_n}{6\Delta t_n},$$

$t_n$ is a nominal period of the periodic strays, $\Delta t_n$ is an admissible tolerance of $t_n$ and $t_{min}=t_n-\Delta t_n$.

4. An averaging method according to claim 2 characterized in that the permanent desynchronization between the external stimuli frequency 1/T and the frequency 1/t of the periodic strays is realized in that the period

$$T=K(t_n+\Delta t_n)+\tfrac{1}{3}(t_n+\Delta t_n)+\tau$$

where

$$\tau=2\Delta t_n(K+\tfrac{1}{3}),$$

and where the phase shift α corresponds to $\frac{1}{3}(t_n+\Delta t_n)+\tau$.

5. An averaging method according to claim 3 characterized in that the permanent desynchronization between the external stimuli frequency 1/T and the frequency 1/t of the periodic strays is realized in that the period

$$T' = K'(t_n - \Delta t_n) + \tfrac{2}{3}(t_n - \Delta t_n) + \tau,$$

where

$$\tau = \tfrac{1}{3}t_n - 2\Delta t_n(K' + \tfrac{1}{2}),$$

and where the phase shift $\alpha$ corresponds to $\tfrac{2}{3}(t_n - \Delta t_n) + \tau$.

6. A circuit arrangement for an evoked response measuring set-up for applying an averaging method for strays elimination comprising a triggering circuit (3) delivering common pulses which trigger successive sweeps of the averaging procedure in an averaging computer (6) and simultaneously trigger external stimuli in transducers (4) controlled by a stimulator (5) characterized in that the triggering circuit (3) is connected through a dividing circuit (2) to a quartz oscillator circuit (1), in that the triggering circuit (3) is driven by pulses of highly stable frequency 1/T, which pulses are supplied by the dividing circuit (2) by dividing the frequency $f_{gz}$ of pulses supplied by the quartz oscillator circuit (1) to the dividing circuit (2) by a chosen integer P, which frequency $f_{gz}$ is calculated from the formula

$$f_{gz} = \frac{P}{(t_n + \Delta t_n)(K + \tfrac{1}{3})},$$

where $t_n$ is a nominal period of the periodic strays, $\Delta t_n$ is an admissible tolerance of $t_n$ and K is an integer within the range

$$0 \leq K \leq \frac{t_n - 5\Delta t_n}{6\Delta t_n}.$$

7. A circuit arrangement for an evoked response measuring set-up for applying an averaging method for strays elimination comprising a triggering circuit (3) delivering common pulses which trigger successive sweeps of the averaging procedure in an averaging computer (6) and simultaneously trigger external stimuli in transducers (4) controlled by a stimulator (5) characterized in that the triggering circuit (3) is connected through a dividing circuit (2) to a quartz oscillator circuit (1), in that the triggering circuit (3) is driven by pulses of highly stable frequency 1/T, supplied by the dividing circuit, which frequency 1/T is determined by dividing the frequency $f'_{qz}$ of pulses supplied by the quartz oscillator circuit (1) to the dividing circuit by a chosen integer P in the dividing circuit (2), which frequency $f'_{gz}$ can be calculated from the formula

$$f'_{gz} = \frac{P}{(t_n - \Delta t_n)(K' + \tfrac{2}{3})},$$

where $t_n$ is a nominal period of the periodic strays, $\Delta t_n$ is an admissible tolerance of $t_n$ and K' is an integer within the range

$$0 < K' < \frac{t_n - 3\Delta t_n}{6\Delta t_n}.$$

**Patentansprüche**

1. Verfahren der Mittelwertbildung zum Beseitigen periodischer Störungen in Analog-Digital-Rechnern mit einer Prozedur der Mittelwertbildung, wobei ein Satz von Signalen $U_{zs}$, die durch Elektroden (10) von einem auf externe Stimuli ansprechenden Prüfsubjekt (8) aufgenommen werden, verarbeitet wird, um ein gemitteltes hervorgerufenes Ansprechsignal $\overline{U_{ow}}$ zu bilden, dadurch gekennzeichnet, daß aus dem Satz von Signalen $U_{zs}$ periodische Störungen $U_{zp}$ während der Prozedur der Mittelwertbildung proportional zur Anzahl N der Durchläufe der Mittelwertbildungs-Prozedur beseitigt werden, wobei eine zumindest N-fache Verringerung der Amplitude der Störungen erreicht wird durch Auslösen der externen Stimuli und gleichzeitig der Durchläufe der Prozedur der Mittelwertbildung zu solchen Zeiten, daß die Phasen der peridoischen Störungen bei zwei aufeinanderfolgenden Auslösungen voneinander abweichen durch eine Phasenverschiebung $\alpha$ innerhalb des Bereiches $120° \leq \alpha \leq 240°$.

2. Verfahren der Mittelwertbildung nach Anspruch 1, dadurch gekennzeichnet, daß die Phasenverschiebung $\alpha$ durch eine permanente Desynchronisierung zwischen den externen Stimuli mit der Frequenz 1/T und den periodischen Störungen mit der Frequenz 1/t sichergestellt wird und die

Desynchronisierung durch $T=Kt_{max}+\frac{1}{3}t_{max}$ bestimmt wird, worin K eine natürliche Zahl innerhalb des Bereiches

$$0 \leq K \leq (t_n - 5\Delta t_n)/6\Delta t_n,$$

$t_n$ eine Nennperiode der periodischen Störungen, $\Delta t_n$ eine zulässige Toleranz von $t_n$ und $t_{max}=t_n+\Delta t_n$ bedeuten.

3. Verfahren der Mittelwertbildung nach Anspruch 1, dadurch gekennzeichnet, daß die Phasenverschiebung $\alpha$ durch eine permanente Desynchronisierung zwischen den externen Stimuli mit der Frequenz 1/T' und den periodischen Störungen mit der Frequenz 1/t sichergestellt wird und die Desynchronisierung durch $T'=K't_{min}+\frac{2}{3}t_{min}$ bestimmt wird, worin K' eine natürliche Zahl innerhalb des Bereiches

$$0 \leq K' \leq (t_n - 3\Delta t_n)/6\Delta t_n,$$

$t_n$ eine Nennperiode der periodischen Störungen, $\Delta t_n$ eine zulässige Toleranz von $t_n$ und $t_{min}=t_n-\Delta t_n$ bedeuten.

4. Verfahren der Mittelwertbildung nach Anspruch 2, dadurch gekennzeichnet, daß die permanente Desynchronisierung zwischen der Frequenz 1/T der externen Stimuli und der Frequenz 1/t der periodischen Störungen dadurch verwirklicht wird, daß die Periode

$$T=K(t_n+\Delta t_n)+\frac{1}{3}(t_n+\Delta t_n)+\tau \text{ ist,}$$

worin $\tau=2\Delta t_n(K+\frac{1}{3})$ ist und die Phasenverschiebung $\alpha$ dem Wert von $\frac{1}{3}(t_n+\Delta t_n)+\tau$ entspricht.

5. Verfahren der Mittelwertbildung nach Anspruch 3, dadurch gekennzeichnet, daß die permanente Desynchronisierung zwischen der Frequenz 1/T' der externen Stimuli und der Frequenz 1/t der periodischen Störungen dadurch verwirklicht wird, daß die Periode

$$T'=K(t_n-\Delta t_n)+\frac{2}{3}(t_n-\Delta t_n)+\tau \text{ ist,}$$

worin

$$\tau=\frac{1}{3}t_n-2\Delta t_n(K'+\frac{1}{2}) \text{ ist}$$

und die Phasenverschiebung $\alpha$ dem Wert von $\frac{2}{3}(t_n-\Delta t_n)+\tau$ entspricht.

6. Schaltungsanordnung für eine Einrichtung zur Messung von hervorgerufenen Ansprechsignalen für die Verwendung eines Verfahrens der Mittelwertbildung zum Beseitigen von Störungen, umfassend einen Auslöseschaltkreis (3), der gemeinsame Impulse liefert, welche aufeinanderfolgende Durchläufe der Prozedur der Mittelwertbildung in einem Mittelwerte bildenden Rechner (6) und gleichzeitig externe Stimuli in Übertragern (4) auslösen, die durch einen Stimulator (5) gesteuert werden, dadurch gekennzeichnet, daß der Auslöseschaltkreis (3) durch einen Teilerschaltkreis (2) mit einem Quarz-Schwingkreis (1) verbunden ist und daß der Auslöseschaltkreis (3) durch Impulse mit der hochstabilen Frequenz 1/T getrieben wird, welche durch den Teilerschaltkreis (2) geliefert werden durch Teilen der Frequenz $f_{gz}$ von Impulsen, die vom Quartz-Schwingkreis (1) an den Teilerschaltkreis (2) geliefert werden, durch eine ausgewählte ganze Zahl P, wobei die Frequenz $f_{gz}$ nach der Formel

$$f_{gz}=P/(t_n+\Delta t_n)(K+\frac{1}{3})$$

berechnet ist, worin $t_n$ eine Nennperiode der periodischen Störungen, $\Delta t_n$ eine zulässige Toleranz von $t_n$ und K eine ganze Zahl aus dem Bereich

$$0 \leq K \leq (t_n - 5\Delta t_n)/6\Delta t_n$$

bedeuten.

7. Schaltungsanordnung für eine Einrichtung zur Messung von hervorgerufenen Ansprechsignalen für die Verwendung eines Verfahrens der Mittelwertbildung zum Beseitigen von Störungen, umfassend einen Auslöseschaltkreis (3), der gemeinsame Impulse liefert, welche aufeinanderfolgende Durchläufe der Prozedur der Mittelwertbildung in einem Mittelwerte bildenden Rechner (6) und gleichzeitig externe Stimuli in Übertragen (4) auslösen, die durch einen Stimulator (5) gesteuert werden, dadurch gekennzeichnet, daß der Auslöseschaltkreis (3) durch einen Teilerschaltkreis (2) mit einem Quarz-Schwingkreis (1) verbunden ist und daß der Auslöseschaltkreis (3) durch Impulse mit der hochstabilen Frequenz 1/T getrieben wird, welche durch den Teilerschaltkreis (2) geliefert werden durch Teilen der Frequenz $f'_{gz}$ von Impulsen, die vom Quarz-Schwingkreis (1) an den Teilerschaltkreis (2) geliefert werden, durch eine ausgewählte ganze Zahl P, wobei die Frequenz $f'_{gz}$ nach der Formel

$$f'_{gz}=P/(t_n-\Delta t_n)(K'+\frac{2}{3})$$

12

berechnet ist, worin $t_n$ eine Nennperiode der periodischen Störungen, $\Delta t_n$ eine zulässige Toleranz von $t_n$ und K' eine ganze Zahl aus dem Bereich

$$0<K'<(t_n-3\Delta t_n)/6\Delta t_n$$

bedeuten.

## Revendications

1. Procédé de calcul de moyenne servant à l'élimination de parasites périodiques dans des calculateurs analogiques-numériques employant un processus de calcul de moyenne, dans lequel procédé un ensemble de signaux $U_{zs}$ captés par des électrodes (10) en provenance d'un objet examiné (8) répondant à des stimuli externes est traité de manière à produire une réponse suscitée moyenne $U_{ow}$, caractérisé en ce qu'on élimine les parasites périodiques $U_{zp}$ de l'ensemble de signaux $U_{zs}$ pendant le processus de calcul de moyenne en proportion du nombre N de passages du processus de calcul de moyenne, en obtenant au moins une réduction d'ordre N de l'amplitude des parasites par déclenchement des stimuli externes et, simultanément, des balayages du processus de calcul de moyenne à des instants tels quelles phases des parasites périodiques relatifs à deux déclenchements successifs diffèrent d'un déphasage a compris dans l'intervalle 120°≦a≦240°.

2. Procédé de calcul de moyenne selon la revendication 1, caractérisé en ce que le déphasage a est fixé par une désynchronisation permanent entre les stimuli externes de fréquence 1/T et les parasites périodiques de fréquence 1/t et ladite désynchronisation est déterminée par $T=Kt_{max}+t_{max}/3$, où K est un entier naturel compris dans l'intervalle

$$0\leqq K\leqq(t_n-5\Delta t_n)/6\Delta t_n,$$

$t_n$ est la période nominale des parasites périodiques, $\Delta t_n$ est la tolérance admissible pour $t_n$, et $t_{max}=t_n+\Delta t_n$.

3. Procédé de calcul de moyenne selon la revendication 1, caractérisé en ce que le déphasage a est fixé par une désynchronisation permanente entre les stimuli externes de fréquence 1/T et les parasites périodiques de fréquence 1/t et ladite désynchronisation est déterminée par

$$T'=K't_{min}+2t_{min}/3,$$

où K' est un entier naturel compris dans l'intervalle

$$0<K'<(t_n-3\Delta t_n)/6\Delta t_n,$$

$t_n$ est la période nominale des parasites périodiques, $\Delta t_n$ est la tolérance admissible pour $t_n$, et $t_{min}=t_n-\Delta t_n$.

4. Procédé de calcul de moyenne selon la revendication 2, caractérisé en ce que la désynchronisation permanente entre la fréquence 1/T des stimuli externes et la fréquence 1/t des parasites périodiques est réalisée par le fait que la période T vaut

$$T=K(t_n+\Delta t_n)+(t_n+\Delta t_n)/3+\tau, \text{ où } \tau=2\Delta t_n(K+\tfrac{1}{3}),$$

et où le déphasage a correspond à $(t_n+\Delta t_n)/3+\tau$.

5. Procédé de calcul de moyenne selon la revendication 3, caractérisé en ce que la désynchronisation permanente entre la fréquence 1/T des stimuli externes et la fréquence 1/t des parasites périodiques est réalisée par le fait que la période T' vaut

$$T'=K'(t_n-\Delta t_n)+2(t_n-\Delta t_n)/3+\tau,$$

où

$$\tau=t_n/3-2\Delta t_n(K'+\tfrac{1}{2}),$$

et où le déphasage a correspond à $2(t_n-\Delta t_n)/3+\tau$.

6. Circuit pour montage de mesure de réponses suscitées permettant d'appliquer un procédé de calcul de moyenne servant à l'élimination de parasites, comprenant un circuit de déclenchement (3) délivrant des impulsions communes qui déclenchent des passages successifs du processus de calcul de moyenne dans un calculateur de calcul de moyenne (6) et, simultanément, déclenchent des stimuli externes dans des transducteurs (4) commandés par un stimulateur (5), caractérisé en ce que le circuit de déclenchement (3) est connecté via un circuit diviseur (2) à un circuit oscillateur à quartz (1), en ce que le circuit de déclenchement (3) est excité par des impulsions de fréquence 1/T hautement stable, lesquelles impulsions sont fournies par le circuit diviseur (2) par division de la fréquence $f_{gz}$ des impulsions fournies par le circuit oscillateur à quartz (1) au circuit diviseur (2) par un entier P choisi, laquelle fréquence $f_{gz}$ est calculée à partir de la formule

$$f_{gz}=P/[(t_n+\Delta t_n)(K+\tfrac{1}{3})],$$

où $t_n$ est la période nominale des parasites périodiques, $\Delta t_n$ est la tolérance admissible pour $t_n$, et K est un entier compris dans l'intervalle

$$0 \leqq K \leqq (t_n - 5\Delta t_n)/6\Delta t_n.$$

7. Circuit pour montage de mesure de réponses suscitées permettant d'appliquer un procédé de calcul de moyenne servant à l'élimination de parasites, comprenant un circuit de déclenchement (3) délivrant des impulsions communes qui déclenchent des passages successifs du processus de calcul de moyenne dans un calculateur de calcul de moyenne (6) et, simultanément, déclenchent des stimuli externes dans des transducteurs (4) commandés par un stimulateur (5), caractérisé en ce que le circuit de déclenchement (3) est connecté via un circuit diviseur (2) à un circuit oscillateur à quartz (1), en ce que le circuit de déclenchement (3) est excité par des impulsions de fréquence 1/T hautement stable, fournies par le circuit diviseur, laquelle fréquence 1/T est déterminée par division de la fréquence $f'_{gz}$ des impulsions délivrées par le circuit oscillateur à quartz (1) au circuit diviseur par un entier P choisi dans le circuit diviseur (2), laquelle fréquence $f'_{gz}$ peut être calculée à partir de la formule

$$f'_{gz} = P/[(t_n - \Delta t_n)(K' + \tfrac{2}{3})],$$

où $t_n$ es la période nominale des parasites périodiques, $\Delta t_n$ est la tolérance admissible pour $t_n$, et K' est un entier compris dans l'intervalle

$$0 < K' < (t_n - 3\Delta t_n)/6\Delta t_n.$$

Fig.1

Fig. 2

dla K'= 5

$$K't_{min}$$

$$\frac{2}{3}t_{min}$$

$$T' = K't_{min} + \frac{2}{3}t_{min}$$

$$T' = K't_{max} + \frac{1}{3}t_{max} + \tau'$$

$$K't_{max}$$

$$\frac{1}{3}t_{max}$$

$$\tau'$$

Fig. 3

EP 0 150 246 B1

Fig. 4

Fig. 5

EP 0 150 246 B1

dla $\alpha$ = 120°

Fig. 6

$A = \frac{a}{1}$

$B = \frac{a+b}{2}$

$C = \frac{a+b+c}{3}$

$D = \frac{a+b+c+d}{4}$

$E = \frac{a+b+c+d+e}{5}$

Fig.7